# EUROPEAN PATENT SPECIFICATION

(11) **EP 0 671 918 B1**
(45) Date of publication and mention of the grant of the patent: **28.05.1997**
(21) Application number: 94900549.0
(22) Date of filing: 03.11.1993
(51) Int. Cl.: A61K 31/70

(54) **COMPOSITIONS CONTAINING ACYCLOVIR-LIKE COMPOUNDS AND 2'-VINYL SUBSTITUTED NUCLEOSIDE ANALOGS FOR THE TREATMENT OF VIRAL INFECTIONS**
ZUBEREITUNGEN, DIE ACYCLOVIR-ÄHNLICHE VERBINDUNGEN UND 2'-VINYLSUBSTITUIERTE NUKLEOSIDANALOGE ENTHALTEN, ZUR BEHANDLUNG VIRALER INFEKTIONEN
COMPOSITIONS CONTENANT DES COMPOSES ANALOGUES A L'ACYCLOVIR ET DES ANALOGUES NUCLEOSIDIQUES SUBSTITUES PAR VINYLE EN POSITION 2' UTILISES DANS LE TRAITEMENT D'INFECTIONS VIRALES

(30) Priority: 03.12.1992 US 984757
(43) Date of publication of application: 20.09.1995
(73) Proprietor: MERRELL PHARMACEUTICALS INC., Cincinnati Ohio 45215 (US)
(72) Inventor: SUNKARA, Sai Prasad, San Diego, CL 92130 (US); McCARTHY, James Ray, Solona Beach, CL 92075 (US)
(74) Representative: Macchetta, Francesco
(86) International application number: US9310649
(87) International publication number: WO9412188

(56) References cited:
- EP-A- 0 143 987
- EP-A- 0 345 751
- EP-A- 0 468 866
- EP-A- 0 477 871
- WO-A-83/02723

## Description

This invention relates to the use of a combination of certain 2'-vinyl substituted nucleoside analogs with acyclovir-like compounds in the treatment of a patient afflicted with a viral infection.

### BACKGROUND OF THE INVENTION

In the past several years, numerous antiviral therapeutic agents have been developed and marketed for use in the treatment of viral infections caused by viruses of the herpes family, i.e. herpes simplex 1 and 2, varicella zoster virus, cytomegalovirus, Epstein-Barr virus, and the like. Effective antiviral therapeutic agents can be characterized by their efficacy in controlling the growth, replication or infectivity of the aforementioned viruses. However, there are numerous mechanisms which can be employed by different antiviral therapeutic agents which can aid in accomplishing these goals.

One well-known antiviral agent is the compound, acyclovir, known by its chemical name 9-(2-hydroxyethoxymethyl)guanine, and disclosed in U.S. Pat. No. 4,199,574. Acyclovir is converted by nucleoside kinases to acyclovir triphosphate (ACV-TP), which then serves as a substrate of viral DNA polymerase because of its close similarity to the natural nucleotide substrate, deoxyguanosine triphosphate (dGTP). It is surmised that when ACV-TP acts as a substrate for the viral DNA polymerase and is subsequently incorporated into the viral DNA chain, it acts as a DNA chain terminator and prevents viral replication.

Synergistic antiviral combinations of certain ribonucleotide reductase inhibitors with acyclovir are disclosed in U.S. Pat. No. 5,021,437. In that reference, the combinations of 2-acetylpyridine 5-[(2-chloroanilino)thiocarbonyl]thiocarbonohydrazone with acyclovir and analogs thereof were found to exhibit synergistic antiviral properties when used as a medical therapy against viral infections, particulary herpes, selected from herpes simplex 1 and 2, varicella zoster virus, cytomegalovirus, and Epstein-Barr virus.

European Patent Application Publication No. 0 372 268, published June 13, 1990, specifically discloses novel 2'-halomethylidene, 2'-ethenylidene and 2'-ethynylcytidine, uridine, and guanosine derivatives as ribonucleotide reductase inhibitors which are useful as anti-viral and anti-neoplastic agents. Specifically, 2'-deoxy-2'-fluoromethylidenecytidine is disclosed.

Similarly, European Patent Application Publication No. 0 310 673, published April 12, 1989, and Takenuki et al., J. Med. Chem., 31, 1063-1064 (1988) disclose 2'-alkylidenepyrimidine nucleoside derivatives, useful as antiviral agents. Specifically, 2'-deoxy-2'-methylidenecytidine is disclosed.

Despite these recent advances, herpes-type viral infections continue to spread world-wide in almost epidemic proportions. Efforts to find cures are continually frustrated. There remains a clear and present need for new and different methods of treatment of these diseases. It is an object of this invention to provide a new, alternative method of treatment for patients afflicted with these viral infections.

### SUMMARY OF THE INVENTION

The present invention relates to a method of treating a patient afflicted with a viral infection comprising administering to the patient an effective antiviral amount of an acyclovir-like compound in conjunctive therapy with an effective antiviral amount of compounds of formula (1) 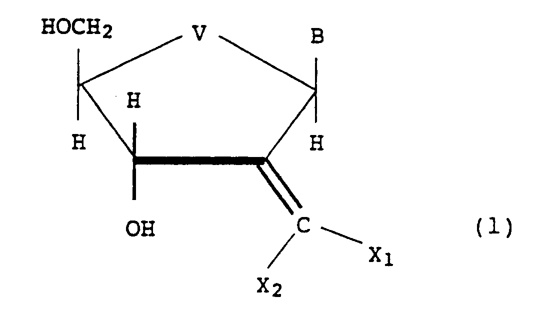 wherein
V is oxy, methylene, or thio,
X₁ and X₂ are each independently hydrogen or halogen,
B is a radical of the formula 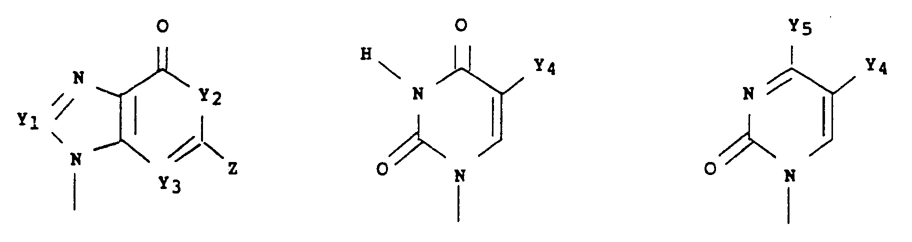
wherein Y₁ is nitrogen, a CH group, a CCl group, a CBr group or a CNH₂ group; Y₂ and Y₃ are each independently nitrogen or a CH group; Y₄ is hydrogen, C₁-C₄ alkyl, C₁-C₄ alkoxy or halogen; Y₅ is amino or C₁-C₄ alkoxy; and Z is hydrogen, halogen, or NH₂;
or a pharmaceutically acceptable salt thereof.

The compounds of formula (1) have been shown to inhibit ribonucleotide reductase and also incorporate in the growing DNA chain causing DNA chain termination.

### DETAILED DESCRIPTION OF THE INVENTION

As used herein, the term "halogen" or "halo-" refers to a fluorine, chlorine, bromine, or iodine atom and the term "nitrogen" refers to a trivalent nitrogen atom attached to two radicals. The term "C₁-C₄ alkyl" refers to a saturated straight or branched chain hydrocarbyl radical of one to four carbon atoms and includes methyl, ethyl, propyl, isopropyl, n-butyl, isobutyl, tertiary butyl and the like. The term "C₁-C₄ alkoxy" refers to a C₁-C₄ alkyl bearing an oxy group and includes methoxy, ethoxy, propoxy, butoxy and the like.

The 2'-vinyl substituted nucleoside analogs contemplated by the compound of formula (1) of this invention include:
(Z) and (E)-2'-deoxy-2'-fluoromethylidenecytidine
(Z) and (E)-2'-deoxy-2'-fluoromethylideneuridine
(Z) and (E)-2'-deoxy-2'-fluoromethylideneguanosine
(Z) and (E)-2'-deoxy-2'-chloromethylidenecytidine
(Z) and (E)-2'-deoxy-2'-chloromethylideneuridine
(Z) and (E)-2'-deoxy-2'-chloromethylideneguanosine and 2'-deoxy-2'-methylidenecytidine.

The preferred compounds according to the present invention are 2'-deoxy-2'-methylidenecytidine and (Z) and (E)-2'-deoxy-2'-fluoromethylidenecytidine. The most preferred is (E)-2'-deoxy-2'-fluoromethylidenecytidine. The compounds of formula (1) may be prepared according to the processes set forth in European Patent Application Publication Number 0 372 268, published June 13, 1990, and European Patent Application Publication Number 0 310 673, published April 12, 1989 which are both incorporated herein by reference as if fully set forth.

Pharmaceutically acceptable salts of the compounds of formula (1) contemplated for use in this invention include those prepared from these corresponding acids: hydrochloric, hydrobromic, sulfuric, nitric, phosphoric, maleic, salicylic, p-toluenesulfonic, tartaric, citric, acetic, methanesulfonic, formic, succinic, naphthalene-2-sulfonic, isethionic, lactobionic and benzenesulfonic. This listing is illustrative only and not intended as a limitation on the scope of this invention. While these salts are acceptable for use in this invention, the free base form of the compounds of formula 1 are preferred.

As used herein, the term "acyclovir-like compound" refers to certain antiviral compounds such as acyclovir, ganciclovir and the like.

The antiviral agent "acyclovir" is a well known antiviral agent, known also by its chemical name of 9-[(2-hydroxyethoxy)methyl]guanine. It is marketed under the brand name Zovirax® by Burroughs Wellcome Co. in the form of capsules, tablets, suspensions, ointments and sterile powders. A method for preparing acyclovir is disclosed in U.S. Pat. No. 4,199,574 which is incorporated by reference herein as if fully set forth.

Pharmaceutically acceptable salts of the acyclovir contemplated for use in this invention include salts of pharmaceutically acceptable organic acids such as lactic, acetic, malic, or p-toluenesulfonic acid as well as salts of pharmaceutically acceptable mineral acids such as hydrochloric or sulfuric acid. This listing is illustrative only and not intended as a limitation on the scope of this invention.

The antiviral agent "ganciclovir" is a well known antiviral agent, known also by its chemical name of 9-(1,3-dihydroxy-2-propoxymethyl)guanine. It is marketed under the brand name Cytovene® by Syntex Laboratories, Inc. in the form of sterile powder for intravenous use only. A method for preparing ganciclovir is disclosed in U.S. Pat. No. 4,507,305 which is incorporated by reference herein as if fully set forth.

Pharmaceutically acceptable salts of the ganciclovir contemplated for use in this invention include acid addition salts and alkali metal salts. Suitable acids for salt formation include inorganic acids such as hydrochloric acid, hydrobromic acid, sulfuric acid, nitric acid, phosphoric acid and the like. Suitable organic acids for salt formation include trifluoroacetic acid, methanesulfonic acid, ethanesulfonic acid, p-toluenesulfonic acid and the like. Appropriate bases for salt formation include alkali metal bases such as alkali metal hydroxides, e.g., sodium hydroxide, potassium hydroxide and the like. This listing is illustrative only and not intended as a limitation on the scope of this invention.

As used herein, the term "patient" refers to a warm-blooded animal such as a mammal which is afflicted with a viral infection. It is understood that dogs, cats, rats, mice, horses, bovine cattle, sheep, and humans are examples of animals within the scope of the meaning of the term.

The term "viral infection" used herein refers to an abnormal state or condition characterized by viral transformation of cells, viral replication and proliferation. Viral infections for which treatment with the method of this invention will be particularly useful include those caused by viruses of the herpes family. Noted examples include herpes simplex virus (HSV) types 1 and 2, varicella zoster virus (VZV), Epstein-Barr virus (EBV), cytomegalovirus (CMV), and the like.

As pertaining to this invention, the term "conjunctive therapy" contemplates co-administration of the compound of formula 1 and the acyclovir-like compound. This co-administration takes place essentially at the same time. Of course, it is understood that the co-administration need not take place at exactly the same time, but should at least be administered on the same day and in amounts that satisfy the daily dosage requirements of each of the compounds.

As used herein, the term "effective antiviral amount" refers to an amount of the compound of formula (1) or the acyclovir-like compound which is effective, upon single or multiple dose administration to the patient, in controlling the growth, replication, or infectivity of the virus or decreasing the amount of virus. As used herein, "controlling the growth" of the virus refers to slowing, interrupting, arresting or stopping the viral transformation of cells or the replication and proliferation of the virus and does not necessarily indicate a total elimination of the virus.

An effective antiviral amount of acyclovir is expected to vary from about 0.1 milligram per kilogram of body weight per day (mg/kg/day) to about 100 mg/kg/day. Preferably from about 5 mg/kg/day to about 50 mg/kg/day of acyclovir is used. Most preferably, an amount of from about 10 mg/kg/day to about 20 mg/kg/day is employed.

An effective antiviral amount of ganciclovir is expected to vary from about 0.1 mg/kg/day to about 100 mg/kg/day. Preferably from about 1 mg/kg/day to about 30 mg/kg/day of ganciclovir is used. Most preferably, an amount of from about 5 mg/kg/day to about 10 mg/kg/day is employed.

An effective antiviral amount of the 2'-vinyl substituted nucleoside analogs of the present invention is expected to vary from about 0.1 mg/kg/day to about 100 mg/kg/day, preferably from about 1 mg/kg/day to about 50 mg/kg/day, and most preferably from about 5 mg/kg/day to about 20 mg/kg/day. However, the most preferred range is expected to vary with the type of 2'-vinyl substituted nucleoside analog involved. For example, the most preferred range of (E)-2'-deoxy-2'-fluoromethylidenecytidine is from about 5 mg/kg/day to about 10 mg/kg/day.

During conjuctive therapy, the preferred ratio of the acyclovir-like compound to the 2'-vinyl substituted nucleoside analog to be used may vary from about 1 to 0.1 to about 1 to 10.

In effecting treatment of a patient afflicted with the above-described viral infection, the acyclovir-like compound may be administered in any form or mode which makes the compound bioavailable in effective amounts. For example, acyclovir-like compounds can be administered orally, subcutaneously, intramuscularly, intravenously, topically, transdermally, intranasally, rectally, and the like. Oral, intravenous or transdermal administration is preferred. One skilled in the art can readily select the proper form and mode of administration depending upon the particular characteristics of the compound selected, the disease state to be treated, and other relevant circumstances.

For infections of external tissues (e.g. the mouth and skin), the acyclovir-like compound may be applied to the infected part of the body of the patient in the form of a topical ointment or cream. For example, the acyclovir-like compound may be presented in an ointment with a water soluble ointment base, or in a cream with an oil in water cream base in a concentration of from about 0.1% to 10%; preferably 0.5% to 7% w/v. An amount of the topical ointment or cream containing the acyclovir-like compound, sufficient to cover the infected part of the body, should be applied in single or multiple applications on a daily basis. The dose size per application will vary depending upon the size of the infected area and the concentration of acyclovir-like compound in the ointment or cream. Preferably, for an ointment or cream having an acyclovir-like compound concentration of about 5% w/v, the dose size should approximate a one-half inch ribbon of ointment per 4 square inches of surface area.

For example, U.S. Pat. No. 4,199,574 states that acyclovir may be given parenterally, orally, used as a suppository or pessary, applied topically as an ointment, cream, aerosol, powder, or given as eye or nose drops, etc., depending on whether the preparation is used to treat internal or external viral infections. Acyclovir is readily available in the form of capsules, tablets, suspensions, oinment (5%), and sterile powder under the trade name Zovirax®, marketed by Burroughs Wellcome Co. Acyclovir is used most preferably in the treatment of a patient afflicted with herpes simplex virus.

Likewise, U.S. Pat. No. 4,507,305 states that ganciclovir may be administered parenterally or orally, although ganciclovir is marketed only as a sterile powder under the trade name Cytovene®, which may only be administered intravenously. For the purposes of this invention, the most preferred use of ganciclovir is its use in conjunctive therapy with a compound of formula (1) for the treatment of a patient afflicted with cytomegalovirus.

The acyclovir-like compound can be administered alone or in the form of a pharmaceutical composition (e.g. Zovirax®) in combination with pharmaceutically acceptable carriers or excipients, the proportion and nature of which are determined by the solubility and chemical properties of the acyclovir-like compound, and standard pharmaceutical practice. Acyclovir-like compounds, while effective themselves, may be formulated and administered in the form of a pharmaceutically acceptable acid addition salt for the purposes of stability, convenience of crystallization, increased solubility and the like.

The 2'-vinyl substituted nucleoside analog may be administered in any form or mode which makes the compound bioavailable in effective amounts, including oral and parenteral routes. For example, it can be administered orally, subcutaneously, intramuscularly, intravenously, topically, transdermally, intranasally, rectally, and the like. Oral administration is generally preferred. One skilled in the art of preparing formulations can readily select the proper form and mode of administration depending upon the particular circumstances, including the viral infection to be treated, the stage of the disease, the form of administration of the acyclovir-like compound, the manner of co-administration selected, and the like.

The 2'-vinyl substituted nucleoside analog can be administered alone or in the form of a pharmaceutical composition in combination with pharmaceutically acceptable carriers or excipients, the proportion and nature of which are determined by the solubility and chemical properties of the 2'-vinyl substituted nucleoside analog and standard pharmaceutical practice. The 2'-vinyl substituted nucleoside analog, while effective itself, may be formulated and administered in the form of its pharmaceutically acceptable acid addition salt, but its free base form is preferred.

In practicing the method of use of the present invention, (Z) and (E)-2'-deoxy-2'-fluoromethylidenecytidine and 2'-deoxy-2'-methylidenecytidine are the preferred 2'-vinyl substituted nucleoside analogs. The most preferred compound of formula (1) is (E)-2'-deoxy-2'-fluoromethylidenecytidine.

The following examples are provided in order to illustrate the method of use of the present invention. These examples are intended to be illustrative only and are not to be construed to limit the scope of the invention in any way.

### EXAMPLE 1

### Effect of Conjunctive Therapy in Vero Cells

Two strains of herpes simplex virus are used: KOS (type 1) and EKC (type 2). Prepare all of the virus stocks by infecting vero cells at an input multiplicity of 0.1 PFU/cell. Sonically disrupt the cultures at 20-24 hours post infection and store them at -70°C after inclusion of DMSO to 5% (v/v) to prevent freeze damage.

Determine the anti-herpes viral activity of the compounds by a known method (Ash and Diekema, Antiviral Research, 8: 71-83 (1987)). For example, infect vero cells with each strain of HSV at 1 PFU/Cell. At the end of the absorption period (60 min., 36°C), remove the unattached virus. Wash cell sheets twice with Eagles basal medium (EBM) and 2% fetal calf serum (FCS). Add fresh medium containing the test compounds. Finally, determine viral yields at 24 hours post infection by plaque method on vero cells with methyl cellulose overlay (0.5% in EBM + 1% FCS).

**Table 1**

| Antiviral Activity of (E)-2'-deoxy-2'-fluoromethylidenecytidine against herpes simplex virus type I | | | |
|---|---|---|---|
| Treatment | Conc. (ug/ml) | Virus Titer P.F.U^{.}^{a}/ml | % Inhibition |
| Viral Control | | 3.7x10⁸ | |
| Compound A^{b} | 0.0125 | 5.5x10⁸ | 0 |
| | 0.025 | 4.5x10⁷ | 88 |
| | 0.050 | 0 | 100 |
| | 0.10 | 0 | 100 |

| | | | |
|---|---|---|---|
| ^{a} P.F.U. = plaque forming units | | | |
| ^{b} Compound A = (E)-2'-deoxy-2'-fluoromethylidenecytidine | | | |

**Table 2**

| Antiviral Activity of (E)-2'-deoxy-2'-fluoromethylidenecytidine against herpes simplex virus type II | | | |
|---|---|---|---|
| Treatment | Conc. (ug/ml) | Virus Titer P.F.U^{.}^{a}/ml | % Inhibition |
| Viral Control | | 1.24x10⁵ | |
| Compound A^{b} | 0.0125 | 8.5x10⁴ | 32 |
| | 0.025 | 5.5x10³ | 96 |
| | 0.050 | 0 | 100 |
| | 0.10 | 0 | 100 |

| | | | |
|---|---|---|---|
| ^{a} P.F.U. = plaque forming units | | | |
| ^{b} Compound A = (E)-2'-deoxy-2'-fluoromethylidenecytidine | | | |

**Table 3**

| Antiviral Activity of conjunctive therapy of (E)-2'-deoxy-2'-fluoromethylidenecytidine and acyclovir against herpes simplex virus type I | | | |
|---|---|---|---|
| Treatment | Conc. (ng/ml) | Virus Titer P.F.U.^{a}/ml | % Inhibition |
| Viral Control | | 2.6x10⁵ | |
| Compound A^{b} (ng/ml) | 5.0 | 1.6x10⁵ | 42 |
| ACV^{c} | 2.5 | 3x10⁵ | 0 |
| | 5.0 | 1.85x10⁵ | 30 |
| ACV (5ng/ml) + Compound A(5ng/ml) | | 8x10⁴ | 70 |
| ACV (2.5ng/ml) + Compound A(5ng/ml) | | 9.5x10⁴ | 64 |

| | | | |
|---|---|---|---|
| ^{a} P.F.U. = plaque forming units | | | |
| ^{b} Compound A = (E)-2'-deoxy-2'-fluoromethylidenecytidine | | | |
| ^{c} ACV = Acyclovir | | | |

## Claims

1. Use of an effective antiviral amount of a 2'-vinyl substituted nucleoside analog compound of the formula 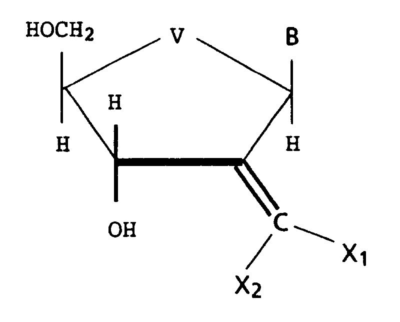 wherein
V is oxy, methylene or thio,
X₁ and X₂ are each independently hydrogen or halogen,
B is a group of the formula 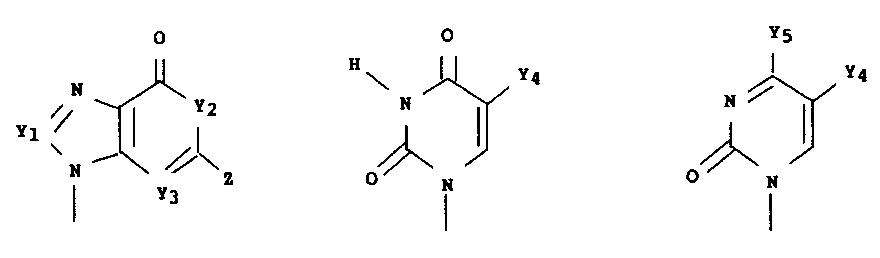
wherein Y₁ is nitrogen, a CH group, a CCl group, a CBr group or a CNH₂ group; Y₂ and Y₃ are each independently nitrogen or a CH group; Y₄ is hydrogen, C₁-C₄ alkyl, C₁-C₄ alkoxy or halogen; Y₅ is amino or C₁-C₄ alkoxy; and Z is hydrogen, halogen or NH₂; or a pharmaceutically acceptable salt thereof,
for the preparation of a medicament for a conjunctive therapy with an acyclovir-like compound.

2. Use according to Claim 1 wherein the acyclovir-like compound is acyclovir.

3. Use according to Claim 2 wherein acyclovir is administered with 2'-deoxy-2'-fluoromethylidenecytidine.

4. Use according to Claim 2 wherein acyclovir is administered with (E)-2'-deoxy-2'-fluoromethylidenecytidine.

5. Use according to Claim 2 wherein acyclovir is administered with 2'-deoxy-2'-methylidenecytidine.

6. Use according to any one of claims 1 to 5 wherein the viral infecting agent is a herpes virus.

7. Use according to any one of claims 1 to 5 wherein the viral infecting agent is a herpes simplex virus.

8. Use according to any one of claims 1 to 5 wherein the viral infecting agent is a varicella zoster virus.

9. Use according to any one of claims 1 to 5 wherein the viral infecting agent is a cytomegalovirus.

10. Use according to any one of claims 1 to 5 wherein the viral infecting agent is an Epstein-Barr virus.

11. A combination comprising a 2'-vinyl substituted nucleoside analog compound of the formula: 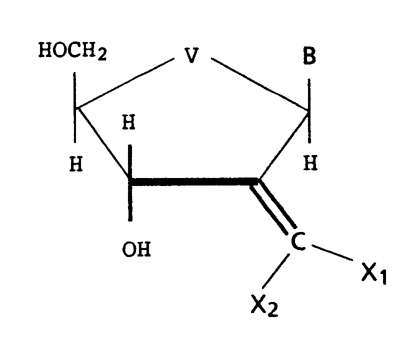 wherein
V is oxy, methylene or thio,
X₁ and X₂ are each independently hydrogen or halogen,
B is a group of the formula 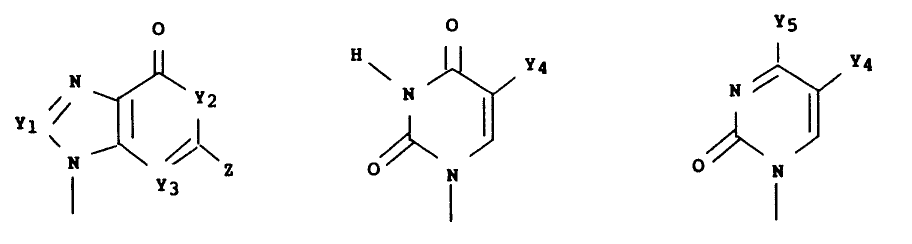
wherein Y₁ is nitrogen, a CH group, a CCl group, a CBr group or a CNH₂ group; Y₂ and Y₃ are each independently nitrogen or a CH group; Y₄ is hydrogen, C₁-C₄ alkyl, C₁-C₄ alkoxy or halogen; Y₅ is amino or C₁-C₄ alkoxy; and Z is hydrogen, halogen, or NH₂; or a pharmaceutically acceptable salt thereof; either alone or in the form of a pharmaceutical composition,
and an acyclovir-like compound, either alone or in the form of a pharmaceutical composition,
for a conjunctive therapy for the treatment of a viral infection.

12. Combination according to Claim 11 wherein the acyclovir-like compound is acyclovir.

13. Combination according to Claim 12 wherein acyclovir is administered with 2'-deoxy-2'-fluoromethylidenecytidine.

14. Combination according to Claim 12 wherein acyclovir is administered with (E)-2'-deoxy-2'-fluoromethylidenecytidine.

15. Combination according to Claim 12 wherein acyclovir is administered with 2'-deoxy-2'-methylidenecytidine.

16. Combination according to any one of claims 11 to 15 wherein the viral infecting agent is a herpes virus, a herpes simplex virus, a varicella zoster virus, a cytomegalovirus, or an Epstein-Barr virus.

17. Combination according to any one of claims 11 to 16 wherein the 2'-vinyl substituted nucleoside analog and the acyclovir-like compound are coformulated in the same pharmaceutical composition.

## Patentansprüche

1. Verwendung einer antiviral wirksamen Menge einer 2'-vinyl-substituierten nucleosidanalogen Verbindung der Formel 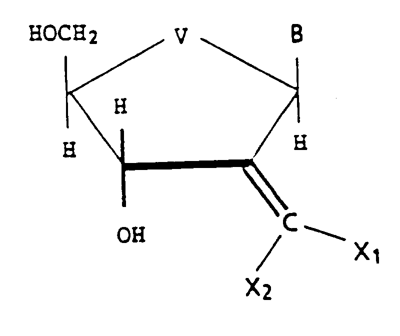 in der
V eine Oxy-, Methylen- oder Thiogruppe ist,
X₁ und X₂ jeweils unabhängig ein Wasserstoff- oder ein Halogenatom sind,
B ein Rest einer der Formeln 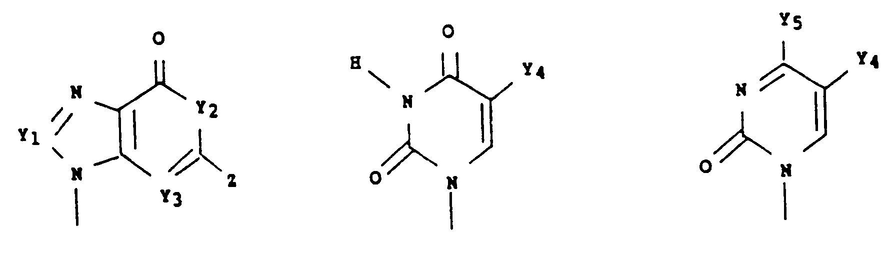
ist, in denen Y₁ ein Stickstoffatom, eine CH-Gruppe, eine CCl-Gruppe, eine CBr-Gruppe oder eine CNH₂-Gruppe ist; Y₂ und Y₃ jeweils unabhängig ein Stickstoffatom oder eine CH-Gruppe sind;
Y₄ ein Wasserstoffatom ein C₁-C₄-Alkylrest, C₁-C₄-Alkoxyrest oder ein Halogenatom ist; Y₅ eine Aminogruppe oder ein C₁-C₄-Alkoxyrest ist und Z ein Wasserstoffatom, Halogenatom oder eine NH₂-Gruppe ist; oder eines pharmazeutisch verträglichen Salzes davon,
zur Herstellung eines Medikaments für eine Kombinationstherapie mit einer acyclovir-ähnlichen Verbindung.

2. Verwendung nach Anspruch 1, wobei die acyclovir-ähnliche Verbindung Acyclovir ist.

3. Verwendung nach Anspruch 2, wobei Acyclovir mit 2'-Desoxy-2'-fluormethylidencytidin verabreicht wird.

4. Verwendung nach Anspruch 2, wobei Acyclovir mit (E)-2'-Desoxy-2'-fluormethylidencytidin verabreicht wird.

5. Verwendung nach Anspruch 2, wobei Acyclovir mit 2'-Desoxy-2'-methylidencytidin verabreicht wird.

6. Verwendung nach einem der Ansprüche 1 bis 5, wobei das viral infizierende Mittel ein Herpes-Virus ist.

7. Verwendung nach einem der Ansprüche 1 bis 5, wobei das viral infizierende Mittel ein Herpes-simplex-Virus ist.

8. Verwendung nach einem der Ansprüche 1 bis 5, wobei das viral infizierende Mittel ein Varicella-Zoster-Virus ist.

9. Verwendung nach einem der Ansprüche 1 bis 5, wobei das viral infizierende Mittel ein Zytomegalievirus ist.

10. Verwendung nach einem der Ansprüche 1 bis 5, wobei das viral infizierende Mittel ein Epstein-Barr-Virus ist.

11. Kombination, umfassend eine 2'-vinyl-substituierte nukleosidanaloge Verbindung der Formel: 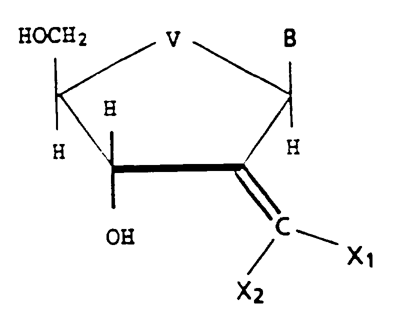 in der
V eine Oxy-, Methylen- oder Thiogruppe ist,
X₁ und X₂ jeweils unabhängig ein Wasserstoff- oder ein Halogenatom sind,
B ein Rest einer der Formeln 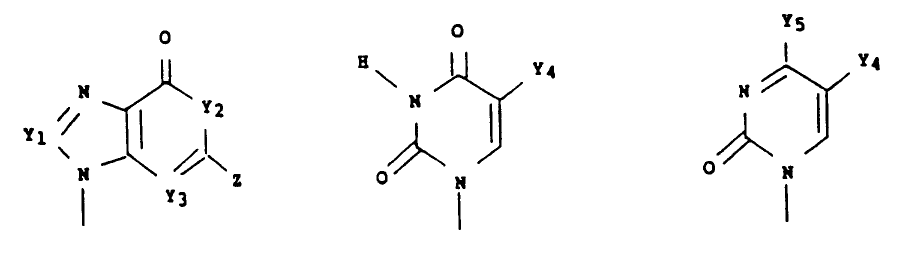
ist, in denen Y₁ ein Stickstoffatom, eine CH-Gruppe, eine CCl-Gruppe, eine CBr-Gruppe oder eine CNH₂-Gruppe ist; Y₂ und Y₃ jeweils unabhängig ein Stickstoffatom oder eine CH-Gruppe sind; Y₄ ein Wasserstoffatom, ein C₁-C₄-Alkylrest, C₁-C₄-Alkoxyrest oder ein Halogenatom ist; Y₅ eine Aminogruppe oder ein C₁-C₄-Alkoxyrest ist und Z ein Wasserstoffatom, Halogenatom oder eine NH₂-Gruppe ist; oder eines pharmazeutisch verträglichen Salzes davon; entweder allein oder in der Form eines Arzneimittels,
und eine acyclovir-ähnliche Verbindung, entweder allein oder in der Form eines Arzneimittels,
für eine Kombinationstherapie einer viralen Infektion.

12. Kombination nach Anspruch 11, wobei die acyclovir-ähnliche Verbindung Acyclovir ist.

13. Kombination nach Anspruch 12, wobei Acyclovir mit 2'-Desoxy-2'-fluormethylidencytidin verabreicht wird.

14. Kombination nach Anspruch 12, wobei Acyclovir mit (E)-2'-Desoxy-2'-fluormethylidencytidin verabreicht wird.

15. Kombination nach Anspruch 12, wobei Acyclovir mit 2'-Desoxy-2'-methylidencytidin verabreicht wird.

16. Kombination nach einem der Ansprüche 11 bis 15, wobei das viral infizierende Mittel ein Herpes-Virus, ein Herpes-simplex-Virus, ein Varicella-Zoster-Virus, ein Zytomegalie-Virus oder ein Epstein-Barr-Virus ist.

17. Kombination nach einem der Ansprüche 11 bis 16, wobei die 2'-vinylsubstituierte nukleosidanaloge und die acyclovir-ähnliche Verbindung in dem gleichen Arzneimittel gemeinsam zubereitet werden.

## Revendications

1. Utilisation d'une quantité antivirale efficace d'un analogue nucléosidique substitué en 2' par vinyle de formule 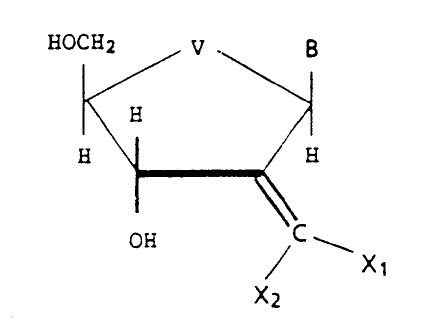 dans laquelle
V est oxy, méthylène, ou thio
X₁ et X₂ sont chacun indépendamment l'atome d'hydrogène ou un halogène
B est un radical de formule 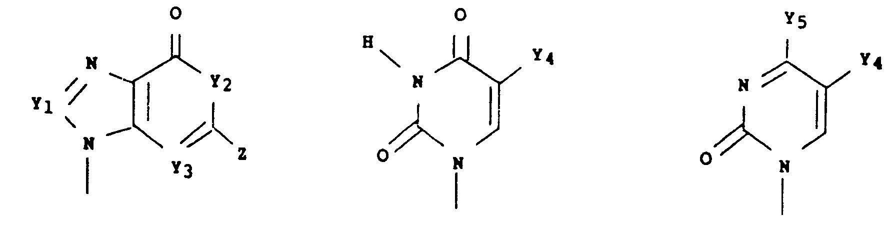
où Y₁ est l'atome d'azote, un groupe CH, un groupe CCl, un groupe CBr ou un groupe CNH₂ ; Y₂ et Y₃ sont chacun indépendamment l'atome d'azote ou un groupe CH ;
Y₄ est l'atome d'hydrogène, alkyle en C₁ - C₄, alcoxy en C₁ - C₄, ou un halogène ; Y₅ est amino ou alcoxy en C₁ - C₄ ; et
Z est l'atome d'hydrogène, un halogène, ou NH₂ ;
ou un de leurs sels acceptables d'un point de vue pharmaceutique,
pour la préparation d'un médicament pour une thérapie adjuvante en association avec un composé de type acyclovir.

2. Utilisation selon la revendication 1, dans laquelle le composé de type acyclovir est l'acyclovir.

3. Utilisation selon la revendication 2, dans laquelle l'acyclovir est administré avec la 2'-désoxy-2'-fluorométhylidènecytidine.

4. Utilisation selon la revendication 2, dans laquelle l'acyclovir est administré avec la (E)-2'-désoxy-2'-fluorométhylidènecytidine.

5. Utilisation selon la revendication 2, dans laquelle l'acyclovir est administré avec la 2'-désoxy-2'-méthylidènecytidine.

6. Utilisation selon l'une quelconque des revendications 1 à 5 dans laquelle l'agent infectieux viral est un virus de l'herpès.

7. Utilisation selon l'une quelconque des revendications 1 à 5, dans laquelle l'agent infectieux viral est un virus de l'herpès simplex.

8. Utilisation selon l'une quelconque des revendications 1 à 5, dans laquelle l'agent infectieux viral est un herpèsvirus varicellae.

9. Utilisation selon l'une quelconque des revendications 1 à 5, dans laquelle l'agent infectieux viral est un cytomégalovirus.

10. Utilisation selon l'une quelconque des revendications 1 à 5, dans laquelle l'agent infectieux viral est le virus d'Epstein-Barr.

11. Association comprenant un analogue nucléosidique substitué en 2' par vinyle de formule 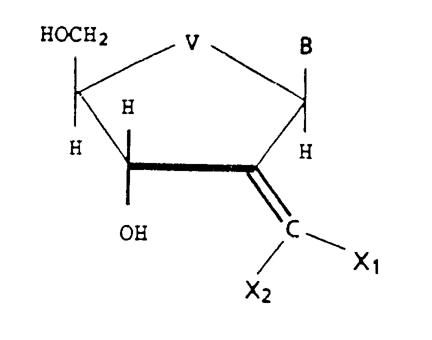 dans laquelle
V est oxy, méthylène, ou thio
X₁ et X₂ sont chacun indépendamment l'atome d'hydrogène ou un halogène
B est un radical de formule 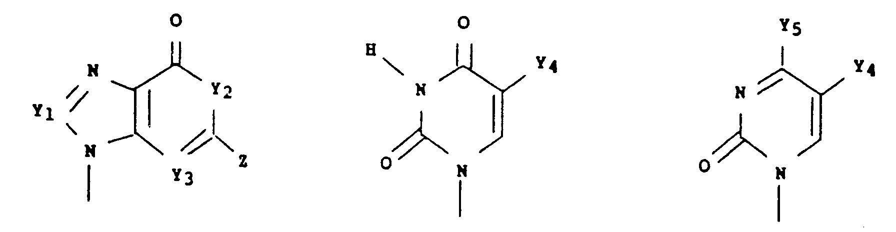
où Y₁ est l'atome d'azote, un groupe CH, un groupe CCl, un groupe CBr ou un groupe CNH₂ ; Y₂ et Y₃ sont chacun indépendamment l'atome d'azote ou un groupe CH ;
Y₄ est l'atome d'hydrogène, alkyle en C₁ - C₄ , alcoxy en C₁ - C₄, ou un halogène ; Y₅ est amino ou alcoxy en C₁ - C₄ ; et
Z est l'atome d'hydrogène, un halogène, ou NH₂ ;
ou un de ses sels acceptable d'un point de vue pharmaceutique, soit seul, soit sous forme de composition pharmaceutique,
et d'un composé de type acyclovir, soit seul, soit sous forme de composition pharmaceutique,
pour une thérapie adjuvante dans le traitement d'une infection virale.

12. Association selon la revendication 11, dans laquelle le composé de type acyclovir est l'acyclovir.

13. Association selon la revendication 12, dans laquelle l'acyclovir est administré avec la 2'-désoxy-2'-fluorométhylidènecytidine.

14. Association selon la revendication 12, dans laquelle l'acyclovir est administré avec la (E)-2'-désoxy-2'-fluorométhylidènecytidine.

15. Association selon la revendication 12, dans laquelle l'acyclovir est administré avec la 2'-désoxy-2'-méthylidènecytidine.

16. Association selon l'une quelconque des revendications 11 à 15, dans laquelle l'agent infectieux viral est un virus de l'herpès, un virus de l'herpès simplex, un herpèsvirus varicellae, un cytomégalovirus, ou un le virus d'Epstein-Barr.

17. Association selon l'une quelconque des revendications 11 à 16, dans laquelle l'analogue nucléosidique substitué en 2' par vinyle et le composé de type acyclovir sont préparés dans la même composition pharmaceutique.
